(19) Europäisches Patentamt
European Patent Office
Office européen des brevets

(11) **EP 3 868 860 B1**

(12) **EUROPEAN PATENT SPECIFICATION**

(45) Date of publication and mention
of the grant of the patent:
**23.04.2025 Bulletin 2025/17**

(21) Application number: **20290024.7**

(22) Date of filing: **21.02.2020**

(51) International Patent Classification (IPC):
**C12M 1/36** (2006.01)

(52) Cooperative Patent Classification (CPC):
**C12M 41/48**

(54) **COMPUTER-IMPLEMENTED METHOD, COMPUTER PROGRAM PRODUCT AND SYSTEM FOR SIMULATING A CELL CULTURE PROCESS**

COMPUTERIMPLEMENTIERTES VERFAHREN, COMPUTERPROGRAMMPRODUKT UND SYSTEM ZUR SIMULATION EINES ZELLKULTURPROZESSES

PROCÉDÉ MIS EN ŒUVRE PAR ORDINATEUR, PRODUIT PROGRAMME INFORMATIQUE ET SYSTÈME DE SIMULATION D'UN PROCÉDÉ DE CULTURE DE CELLULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**25.08.2021 Bulletin 2021/34**

(73) Proprietor: **Sartorius Stedim Data Analytics AB 903 33 Umeå (SE)**

(72) Inventors:
• **CLOAREC, Olivier**
 **33200 Bordeaux (FR)**
• **MCCREADY, Christopher**
 **Ontario N6C IX7 (CA)**

(74) Representative: **Novagraaf International SA Chemin de l'Echo 3 1213 Onex, Geneva (CH)**

(56) References cited:
• **SARANTOS KYRIAKOPOULOS ET AL: "Kinetic Modeling of Mammalian Cell Culture Bioprocessing: The Quest to Advance Biomanufacturing", BIOTECHNOLOGY JOURNAL, 14 November 2017 (2017-11-14), DE, pages 1700229, XP055455678, ISSN: 1860-6768, DOI: 10.1002/biot.201700229**

• **PENNY DORKA ET AL: "Metabolic flux-based modeling of mAb production during batch and fed-batch operations", BIOPROCESS AND BIOSYSTEMS ENGINEERING, SPRINGER, BERLIN, DE, vol. 32, no. 2, 17 June 2008 (2008-06-17), pages 183 - 196, XP019716884, ISSN: 1615-7605**

• **CRAVEN STEPHEN ET AL: "Glucose concentration control of a fed-batch mammalian cell bioprocess using a nonlinear model predictive controller", JOURNAL OF PROCESS CONTROL, OXFORD, GB, vol. 24, no. 4, 21 March 2014 (2014-03-21), pages 344 - 357, XP029027420, ISSN: 0959-1524, DOI: 10.1016/ J.JPROCONT.2014.02.007**

• **LIU YANG ET AL: "Bioprocess optimization under uncertainty using ensemble modeling", JOURNAL OF BIOTECHNOLOGY, ELSEVIER, AMSTERDAM, NL, vol. 244, 27 January 2017 (2017-01-27), pages 34 - 44, XP029919826, ISSN: 0168-1656, DOI: 10.1016/J.JBIOTEC.2017.01.013**

EP 3 868 860 B1

## Description

The application relates to a computer-implemented method, a computer program product and a system for simulating and/or controlling a cell culture process.

## BACKGROUND

Cell growth in a cell culture process may be described by a mathematical model. For example, the growth kinetics of cell culture processes may be described using Monod growth kinetics. A model of a cell culture process may be used for simulating the cell culture process. In some circumstances, the simulation results may be used for controlling the cell culture process to obtain desired cell growth in the cell culture process.

When modeling different types and/or phases of cell culture processes, separate models or separate sets of model parameters (e.g. model coefficients) corresponding to the respective types and/or phases of the cell culture processes may be constructed. For instance, a cell culture process under a batch / fed-batch operation may require a model different from a model for a cell culture process under a continuous, media exchange operation. Further, for example, different phases in a fed-batch operation, e.g., an exponential growth phase and a stationary phase, may require different models or at least different sets of model parameters.

Further, the following documents may be considered as disclosing the background art.

SARANTOS KYRIAKOPOULOS ET AL: "Kinetic Modeling of Mammalian Cell Culture Bioprocessing: The Quest to Advance Biomanufacturing", BIOTECHNOLOGY JOURNAL, November 14, 2017, ISSN: 1860-6768, DOI: 10.1002/biot.201700229 relates to mammalian bioprocess relevant kinetic models, in which the simple unstructured-unsegregated approach utilizing empirical Monod type kinetics based on limiting substrates and inhibitory metabolites is commonly used due to the traceability and simple formalism.

PENNY DORKA ET AL: "Metabolic flux-based modeling of mAb production during batch and fed-batch operations", BIOPROCESS AND BIOSYSTEMS ENGINEERING, SPRINGER, BERLIN, DE, vol. 32, no. 2, June 17, 2008, pages 183-196 discloses mathematical models that predict the physiology, growth behavior and productivity of hybridoma cells in both batch and fed-batch systems.

CRAVEN STEPHEN ET AL: "Glucose concentration control of a fed-batch mammalian cell bioprocess using a nonlinear model predictive controller", JOURNAL OF PROCESS CONTROL, OXFORD, GB, vol. 24, no. 4, 21 March 2014 (2014-03-21), pages 344-357, XP029027420 ISSN: 0959-1524, DOI: 10.1016/J.JP ROCONT.2014.02.007 discloses non-linear model predictive controller (NMPC) for controlling the bioreactor environment in a Chinese hamster ovary (CHO) mammalian cell fed-batch process. The NMPC involves a nonlinear fundamental bioprocess model to represent the CHO mammalian cell fed-batch bioprocess under study.

LIU YANG ET AL: "Bioprocess optimization under uncertainty using ensemble modeling", JOURNAL OF BIOTECHNOLOGY, ELSEVIER, AMSTERDAM, NL, vol. 244, 27 January 2017 (2017-01-27), pages 34-44, ISSN: 0168-1656, DOI: 10.1016/J.JBIOTEC.2017.01.013 ensemble modelling to account for model uncertainty in bioprocess optimization, which adopts a Bayesian approach to define the posterior distribution of the model parameters. Based on the posterior distribution of the model parameter, an ensemble of model parameters may be generated using a uniformly distributed sampling of the parameter confidence region. The ensemble-based process optimization involves maximizing the lower confidence bound of the desired bioprocess objective (e.g. yield or product titer), using a mean-standard deviation utility function.

## SUMMARY

According to an aspect, the problem relates to providing a reliable model of a cell culture process that is applicable to different types and/or phases of cell culture processes, thereby facilitating control of the cell culture process.

This problem is solved by the features disclosed by the independent claims. Further exemplary embodiments are defined by the dependent claims.

According to an aspect, a computer-implemented method for simulating a cell culture process is provided. The method includes:

   receiving one or more measurable parameter values relating to one or more operating conditions of the cell culture process, the one or more operating conditions including one or more of: fresh media feed flow rate, fresh media composition, cell bleed flow rate, harvest flow rate, temperature, pH, dissolved oxygen, agitation rate;
   simulating the cell culture process using:

      a model of the cell culture process;
      estimated values of unmeasurable parameters in the model; and

the received one or more measurable parameter values;

receiving information indicating desired cell growth in the cell culture process; and
determining, according to a result of said simulating the cell culture process, optimal operating conditions for obtaining the desired cell growth.

In the method according to the above-stated aspect, the model of the cell culture process describes the cell culture process with coupled ordinary differential equations including measurable parameters and the unmeasurable parameters, one or more of the measurable parameters relating to said one or more operating conditions of the cell culture process. Further, one or more of the unmeasurable parameters relate to lysed cells in the cell culture process. Said one or more of the unmeasurable parameters include concentration of the lysed cells in the cell culture process and/or concentration of a biomaterial that has a toxic influence on the viable cells. The concentration of the lysed cells is tracked through modeling disintegration of dead cells and production of a toxic biomaterial is modeled as a function of viable cell density. Moreover, the modeling of the disintegration of the dead cells involves cell death rate that is adjusted by the concentration of the lysed cells and/or concentration of the toxic biomaterial. Further, the estimated values of the unmeasurable parameters are estimated using Bayesian inference with measurable parameter values that are measured with respect to at least one operation of the cell culture process.

The method according to the above stated aspect may further include:

obtaining the measurable parameter values that are measured with respect to the at least one operation of the cell culture process;
estimating, using Bayesian inference with the obtained measurable parameter values, values of unmeasurable parameters in the model to be used as the estimated values of the unmeasurable parameters in said simulating the cell culture process.

In the present disclosure, the term "measurable parameter" may indicate a parameter, the value of which can be measured and/or quantified with respect to an operation of a cell culture process. For example, a value of a measurable parameter may be measured with an appropriate sensor during an operation of a cell culture process. Further, for example, a value of a measurable parameter may be quantified based on a calculation using values measured with one or more sensors during an operation of a cell culture process.

Examples of the measurable parameters in the present disclosure may include, but are not limited to, fresh media feed flow rate, fresh media metabolite concentrations, cell bleed flow rate, harvest flow rate, recycle flow rate, bulk solution volume, viable cell density, viability (e.g. percentage of live viable cells), visible dead cell concentration, concentration of a metabolite, temperature, pH, dissolved oxygen, etc.

Further, in the present disclosure, the term "unmeasurable parameter" may indicate a parameter, the value of which cannot be directly measured and/or quantified in the manner analogous to that for measuring and/or quantifying a value of a measurable parameter as stated above.

Further, in the present disclosure, "lysed cells" may be understood as dead cells that have disintegrated and become part of the bulk fluid.

Further examples of the unmeasurable parameters in the present disclosure may include, but are not limited to, maximum growth rate, death rate of cells in the absence of lysed cells, toxicity of lysed cells or a biomaterial, a substrate value below which cell growth is inhibited, sensitivity to deviation from quadratic terms from optimal conditions, a value of inhibition terms above which growth is inhibited, etc.

In various embodiments and examples described herein, as also stated above, the "operating conditions" of the cell culture process include one or more of: fresh media feed flow rate, fresh media composition, cell bleed flow rate, harvest flow rate, temperature, pH, dissolved oxygen, agitation rate. The "operating conditions" may be understood to be directly measured or specified. For instance, a setpoint may be specified as a target value for a parameter representing an operating condition.

As also stated above, in various embodiments and examples described herein, the modeling of the disintegration of the dead cells involves cell death rate that is adjusted by the concentration of the lysed cells and/or concentration of the toxic biomaterial.

In some embodiments and examples, the coupled ordinary differential equations may include the following equations (4) and (5) that relate to the modeling of the disintegration of the dead cells:

$$\frac{dx_l}{dt} = k_l x_d - \frac{(F_h + F_b)}{V} x_l \qquad (4);$$

and

$$\frac{dx_d}{dt} = \left(\mu_d - \frac{F_b}{V}\right) x_d - k_l x_d \qquad (5),$$

wherein $x_l$ is the concentration of the lysed cells, $k_l$ is the rate of dead cells transitioning to the lysed cells, $x_d$ is dead cell concentration, $F_h$ is harvest flow rate of the cell culture process, $F_b$ is cell bleed flow rate of the cell culture process, $V$ is bulk solution volume of the cell culture process and $\mu_d$ is cell death rate.

The cell death rate $\mu_d$ may be defined by the following equation (9):

$$\mu_d = k_d + k_t \varphi_t \qquad (9).$$

wherein $k_d$ is death rate in the absence of the lysed cells, $\varphi_t$ is a value of a toxin, which may include $x_l$, concentration m of a metabolite, or a value $\varphi_b$ of a biomaterial, and $k_t$ is a constant representing increase in death rate from the toxin.

Further, as also stated above, the method according to the above stated aspect comprises, among other steps specified above:

receiving information indicating desired cell growth in the cell culture process; and
determining, according to a result of said simulating the cell culture process, optimal operating conditions for obtaining the desired cell growth.

In some circumstances, the determined optimal operating conditions may be provided for display to a user. The user may then operate one or more devices that carry out the cell culture process so that the cell culture process will be carried out under the determined operating conditions. Such a display and the operation by the user may provide an improved, continued man-machine interaction in operating said one or more devices.

Further, the method according to the above stated aspect may further comprise:

generating one or more control signals for controlling one or more devices that carry out the cell culture process to operate under the determined optimal operating conditions; and
outputting the generated one or more control signals.

In case of generating and outputting the one or more control signals as stated above, the method according to the above stated aspect may also be considered as a method for controlling the cell culture process.

According to yet another aspect, a computer program product is provided. The computer program product comprises computer-readable instructions that, when loaded and run on a computer, cause the computer to perform the method according to any one of the above stated aspect and variations thereof.

According to yet another aspect, a system for simulating a cell culture process is provided. The system comprises:

a storage medium storing a model of the cell culture process, the model describing the cell culture process with coupled ordinary differential equations including measurable parameters and unmeasurable parameters, wherein one or more of the measurable parameters relate to one or more operating conditions of the cell culture process and one or more of the unmeasurable parameters relate to lysed cells in the cell culture process, the one or more operating conditions including one or more of: fresh media feed flow rate, fresh media composition, cell bleed flow rate, harvest flow rate, temperature, pH, dissolved oxygen, agitation rate; and
a processor configured to:

receive one or more measurable parameter values relating to said one or more operating conditions of the cell culture process;
simulate the cell culture process using:

the model of the cell culture process;
estimated values of the unmeasurable parameters in the model, wherein the estimated values of the unmeasurable parameters are estimated using Bayesian inference with measurable parameter values that are measured with respect to at least one operation of the cell culture process; and
the received one or more measurable parameter values

receive information indicating desired cell growth in the cell culture process; and
determine, according to a result of said simulating the cell culture process, optimal operating conditions for obtaining the desired cell growth.

In the system according to the above stated aspect, said one or more of the unmeasurable parameters include concentration of the lysed cells in the cell culture process and/or concentration of a biomaterial that has a toxic influence on the viable cells.

In the system according to the above stated aspect, the concentration of the lysed cells is tracked through modeling disintegration of dead cells.

In the system according to the above stated aspect, production of a toxic biomaterial is modeled as a function of viable cell density.

In the system according to the above stated aspect, the modeling of the disintegration of the dead cells involves cell death rate that is adjusted by the concentration of the lysed cells and/or concentration of the toxic biomaterial.

In some embodiments and examples, the coupled ordinary differential equations may include the following equations (4) and (5) that relate to the modeling of the disintegration of the dead cells:

$$\frac{dx_l}{dt} = k_l x_d - \frac{(F_h + F_b)}{V} x_l \qquad (4);$$

and

$$\frac{dx_d}{dt} = \left(\mu_d - \frac{F_b}{V}\right) x_d - k_l x_d \qquad (5),$$

wherein $x_l$ is the concentration of the lysed cells, $k_l$ is the rate of dead cells transitioning to the lysed cells, $x_d$ is dead cell concentration, $F_h$ is harvest flow rate of the cell culture process, $F_b$ is cell bleed flow rate of the cell culture process, V is bulk solution volume of the cell culture process and $\mu_d$ is cell death rate.

The cell death rate $\mu_d$ may be defined by the following equation (9):

$$\mu_d = k_d + k_t \varphi_t \qquad (9).$$

wherein $k_d$ is death rate in the absence of the lysed cells, $\varphi_t$ is a value of a toxin, which may include $x_l$, concentration $m$ of a metabolite, or a value $\varphi_b$ of a biomaterial, and $k_t$ is a constant representing increase in death rate from the toxin. In the system according to the above stated aspect, the processor may be further configured to:

obtain the measurable parameter values that are measured with respect to the at least one operation of the cell culture process; and

estimate, using Bayesian inference with the obtained measurable parameter values, values of the unmeasurable parameters in the model to be used as the estimated values of the unmeasurable parameters in said simulating the cell culture process.

In the system according to the above stated aspect, the processor may be further configured to:

generate one or more control signals for controlling one or more devices that carry out the cell culture process to operate under the determined optimal operating conditions; and

output the generated one or more control signals.

The subject matter described in the application can be implemented as a method or as a system, possibly in the form of one or more computer program products. The subject matter described in the application can be implemented in a data signal or on a machine readable medium, where the medium is embodied in one or more information carriers, such as a CD-ROM, a DVD-ROM, a semiconductor memory, or a hard disk. Such computer program products may cause a data processing apparatus to perform one or more operations described in the application.

In addition, subject matter described in the application can also be implemented as a system including a processor, and a memory coupled to the processor. The memory may encode one or more programs to cause the processor to perform one or more of the methods described in the application. In some examples, the system may be a general purpose computer system. In other examples, the system may be a special purpose computer system including an embedded system.

In some circumstances, any one of the above stated aspects as well as any one of various embodiments and examples described herein may provide one or more of the following advantages:

- providing a single upstream bioprocess model that describes cellular growth profiles over multiple regimes including:

  - exponential growth, stationary and death phases of fed-batch operation, and
  - media exchange operation such as perfusion and intensified processes;

- implementing a Bayesian approach to parameter identification that is:

  - a robust global optimization method for identification of highly correlated kinetic coefficients and
  - statistical methods for model validation, estimates of kinetic coefficient uncertainty and uncertainty in predictive ability.

The fields of use for various aspects, embodiments and examples described herein may include, but are not limited to, process development, process optimization, process simulation (e.g., digital twin), process monitoring and advanced control of upstream bioprocesses. At a high level, in some circumstances, an application according to any one of various aspects, embodiments and examples described herein may be developed into products that provide automated identification of system level bioprocess growth models. In addition, process understanding may be generated as the system coefficients have physical interpretation for improved understanding of growth kinetics.

## BRIEF DESCRIPTION OF THE DRAWINGS

Details of one or more implementations are set forth in the exemplary drawings and description below. Other features will be apparent from the description, the drawings, and from the claims. It should be understood, however, that even though embodiments are separately described, single features of different embodiments may be combined to further embodiments.

Fig. 1 shows a schematic diagram of an exemplary cell culture process system.

Fig. 2 shows an exemplary flow for simulating a cell culture process.

Fig. 3 shows an exemplary flow of Bayesian model parameters and unmeasurable state estimation.

Fig. 4 shows an exemplary hardware configuration of a computer that may be used to implement at least a part of the system described herein.

## DETAILED DESCRIPTION OF EMBODIMENTS

In the following text, a detailed description of examples will be given with reference to the drawings. It should be understood that various modifications to the examples may be made. In particular, one or more elements of one example may be combined and used in other examples to form new examples.

### System Configuration

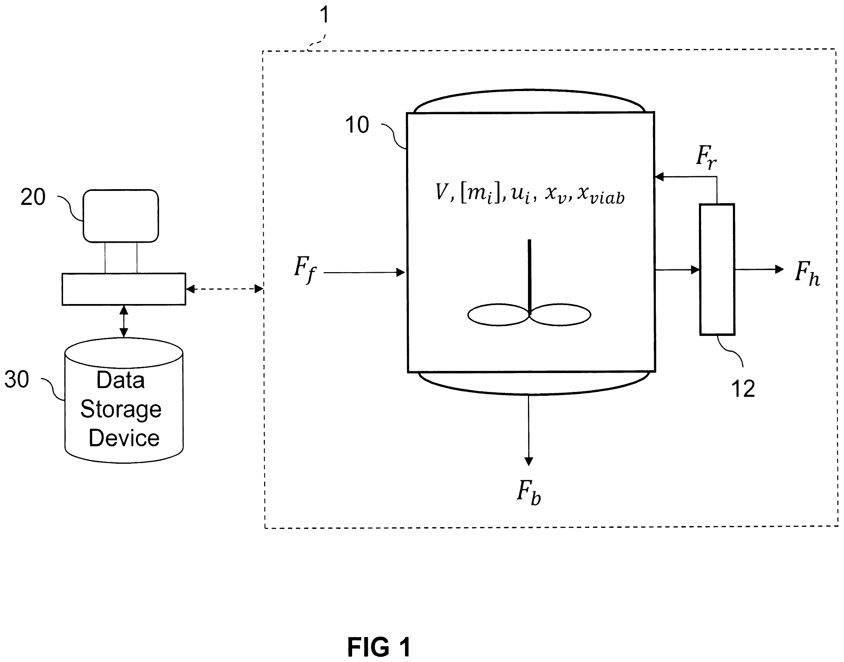

**Fig. 1** shows a schematic diagram of an exemplary cell culture process system. The exemplary system shown in Fig. 1 includes an upstream process system 1, a computing device 20 and a data storage device 30.

The upstream process system 1 may comprise a bioreactor 10 and a media exchange component 12. A cell culture process may take place in the bioreactor 10. The media exchange component 12 may separate bulk fluid from cells in a cell free harvest stream and a cell bleed stream. The media exchange component 12 may be used to collect the desired biological components as harvest material from the bioreactor 10 using at least one separation method.

The computing device 20 may be connected to the upstream process system 1 via (a) wired and/or wireless communication network(s). The computing device 20 may obtain data regarding operations of the upstream process system 1. For example, the computing device 20 may receive one or more values measured by one or more sensors provided in the upstream process system 1. Further, the computing device 20 may provide one or more control signals to control the upstream process system 1. The computing device 20 may be configured to perform a method according to various embodiments and examples described herein. For example, the computing device 20 may be configured to identify a model of a cell culture process carried out by the upstream process system 1 and to simulate the cell culture process, as will be described below. Further, for example, the computing device 20 may be configured to control the upstream process system 1 according to a result of the simulation of the cell culture process. The storage device 30 may store information that is used by the computing device 20 and/or information that is generated by the computing device 20.

**EP 3 868 860 B1**

**Cell Culture Process Model**

The growth kinetics of cell culture processes may be described using Monod growth kinetics, such as those described in equations (1) through (12).

$$x_t = x_v + x_d + x_l \tag{1}$$

$$x_{viab} = \frac{x_v}{x_d + x_v} \tag{2}$$

$$\frac{dx_v}{dt} = \left(\mu_{eff} - \mu_d - \frac{F_b}{V}\right)x_v \tag{3}$$

$$\frac{dx_l}{dt} = k_l x_d - \frac{(F_h + F_b)}{V}x_l \tag{4}$$

$$\frac{dx_d}{dt} = \left(\mu_d - \frac{F_b}{V}\right)x_d - k_l x_d \tag{5}$$

$$\frac{d\varphi_b}{dt} = k_b x_v - \frac{F_f}{V}\varphi_b \tag{6}$$

$$\varphi_t = \varphi_b \ or \ x_l \ or \ m_i \tag{7}$$

$$\mu_{eff} = \mu_{max}\theta_{sub}\theta_{quad}\theta_{inh} \tag{8}$$

$$\mu_d = k_d + k_t\varphi_t \tag{9}$$

$$\theta_{sub} = \prod\left[tanh\left(2\frac{s_i}{\theta_{s,i}}\right)\right]^2 \tag{10}$$

$$\theta_{quad} = \prod exp\left[-\frac{\left(q_i - \theta_{i,opt}\right)^2}{\theta_{q,i}}\right] \tag{11}$$

$$\theta_{inh} = \left[\left(^{I_i}/_{k_i}\right)^3 + 1\right]^{-1} \tag{12}$$

$$\frac{dm_i}{dt} = \delta_i(t)x_v - \frac{(F_h + F_b)}{V}m_i + \frac{F_f}{V}m_{f,i} \tag{13}$$

In the above equations (1) to (13),

7

- $x_t$ is total cells concentration,
- $x_v$ is viable cell concentration,
- $x_d$ is dead cell concentration,
- $x_l$ is concentration of lysed cells,
- $x_{viab}$ is viability (e.g. percent of live visible cells),
- $\mu_{eff}$ is effective cell growth rate,
- $\mu_d$ is cell death rate,
- $F_b$ is cell bleed flow rate (e.g. containing the same material as the material in the vessel, cells and bulk fluid),
- $V$ is bulk solution volume,
- $k_l$ is cell lyse rate (e.g. the rate dead cells transition to lysed cells),
- $F_h$ is harvest flow rate (e.g. bulk fluid with cells separated),
- $\mu_{max}$ is maximum cell growth rate,
- $\varphi_b$ is a value of a biomaterial,
- $k_b$ is generation rate of a biomaterial,
- $\varphi_t$ is a value of a toxin, which may include $x_l$, $m$, or $\varphi_b$,
- $k_t$ is a constant representing the increase in death rate from the toxin,
- $s_i$ is a value of the i$^{th}$ substrate, which could be a state ($x$, $m$) or an independent variable ($u$), where $x$ may be $x_t$, $x_v$, $x_d$, $x_l$ and/or $x_{viab}$, $m$ may be concentration of a metabolite (e.g. $m_i$ and/or $m_{f,i}$ as stated below), and $u$ may relate to one or more operating conditions,
- $\theta_{s,i}$ is a coefficient of the i$^{th}$ substrate below which growth is inhibited,
- $q_i$ is an i$^{th}$ quadratic parameter, which could be a state ($x$, $m$) or independent variable ($u$),
- $\theta_{i,opt}$ is a value of $q_i$ where growth is not inhibited,
- $\theta_{q,i}$ is a coefficient describing the amount of inhibition in growth as $q_i$ deviates from $\theta_{i,opt}$,
- $I_i$ is a value of the i$^{th}$ inhibition parameter, which could be a state ($x$, $m$) or independent variable ($u$),
- $k_i$ is a coefficient above which growth is inhibited,
- $m_i$ is concentration of an i$^{th}$ metabolite,
- $\delta_i(t)$ is specific consumption / secretion rate of the i$^{th}$ metabolite at the current time,
- $F_f$ is fresh media flow rate, and,
- $m_{f,i}$ is concentration of the i$^{th}$ metabolite in $F_f$.

Of the above stated parameters used in the equations (1) to (13), the viable cell concentration $x_v$, the dead cell concentration $x_d$, the viability $x_{viab}$, the cell bleed flow rate $F_b$, the bulk solution volume $V$, the harvest flow rate $F_h$, the value $\varphi_b$ of a biomaterial, the value $s_i$ of the i$^{th}$ substrate, the i$^{th}$ quadratic parameter $q_i$, the value $I_i$ of the i$^{th}$ inhibition parameter, the concentration $m_i$ of an i$^{th}$ metabolite, the specific consumption / secretion rate $\delta_i(t)$ of the i$^{th}$ metabolite at the current time, the fresh media flow rate $F_f$, the concentration $m_{f,i}$ of the i$^{th}$ metabolite in the fresh media flow may be considered as examples of the "measurable parameters" of the present disclosure.

Further, the concentration $x_l$ of the lysed cells, the effective cell growth rate $\mu_{eff}$, the cell death rate $\mu_d$, the cell lyse rate $k_l$, the maximum cell growth rate $\mu_{max}$, generation rate $k_b$ of a biomaterial, the value $\varphi_t$ of the toxin, the constant $k_t$ representing the increase in the death rate from the toxin, the coefficient $\theta_{s,i}$ of the i$^{th}$ substrate below which growth is inhibited, the value $\theta_{i,opt}$ of $q_i$ where growth is not inhibited, the coefficient $\theta_{q,i}$ describing the amount of inhibition in growth as $q_i$ deviates from $\theta_{i,opt}$, the coefficient $k_i$ above which growth is inhibited may be considered as examples of the "unmeasurable parameters" of the present disclosure.

In some circumstances, the value $\varphi_b$ of a biomaterial, listed above as one of the exemplary "measurable parameters", may be dealt with as one of the "unmeasurable parameters". The value $\varphi_b$ of a biomaterial may be observable by its influence when used as a toxin.

Alternatively to the equation (8) that describes the effective growth rate, $\mu_{eff}$, the effective growth rate may be described with by a data driven approach, such as a partial least square (PLS) regression model or a machine learning (ML) model of the following form.

$$\mu_{eff} = f_{\mu_{eff}}\left(x, m, u, \delta(t), \mu_{max}, \varphi, \theta_{sub}, \theta_{quad}, \theta_{inh}\right) \quad (14)$$

In equation (14), $f_{\mu_{eff}}$ may be a data driven type of regression function such as PLS or ML and $\delta(t)$ are the specific consumption / production rates of selected metabolites at the current time.

Often the product concentration or other quality type metrics may be included in the kinetic model. These types of metrics may be modeled using eq. (15).

$$\frac{d\gamma_i}{dt} = k_{\gamma_i} x_v - \frac{F_b + F_h}{V}\gamma_i \qquad (15)$$

$$k_{\gamma_i} = f_{\gamma_i}\big(x, m, u, \delta(t), \mu_{max}, \varphi, \theta_{sub}, \theta_{quad}, \theta_{inh}\big) \qquad (16)$$

In equation (15), $\gamma_i$ is the i$^{th}$ biomaterial, which may be the target protein or quality metric function and the function ($f_{\gamma i}$) is a PLS, Orthogonal Projection on Latent Structure (OPLS), ML or other suitable regression algorithm.

The above equations may be considered as system equations for a cell culture process for fed batch or media exchange. The cell culture process may be carried out by a system shown in Fig. 1, for example.

The parts of the system equations that are unique to the present disclosure may be the way that lysed cells are modeled in equations (4) and (5), as well as the handling of the bulk fluid toxicity in equation (9). Lysed cells are dead cells that have disintegrated and become part of the bulk fluid. Lysed cells are known to exist but not tracked due to the lack of measurements to quantify their concentration. A key enabler of including lysed cells in the system may be a robust global optimization method, which the Bayesian identification method can provide, where there may be enough information about the material balance between live, dead and lysed cells from typical process measurements, which include the viable cell density (VCD) and cell viability.

A second unique aspect of the present disclosure may be the modeling of toxicity. A challenge in current state of the art modeling of batch or fed-batch processes over the duration of their operation may be the change in cellular operation as the batch progresses. In the present disclosure, accumulation of various biomaterials, including lysed cells, metabolites and biomaterials ($x_l$, m, and $\varphi$) may be modeled and treated as toxins to influence the cell death rate and alternatively to influence the growth rate. This may allow for identification of a single model that describes behavior over the complete batch (e.g. fed-batch) cycle.

Modern biomanufacturing processes may often include media exchange (e.g., intensified and perfusion type processing). Media exchange may involve replacing a portion of spent bulk media (e.g., including the toxins as described above) with fresh media. Currently, modeling of media exchange processes may require identification of separate model coefficients for each type of operation. The inclusion of the toxic influence of accumulated biomaterials can allow for transfer of a model calibrated on batch / fed-batch operation to media exchange and ultimately allow for modeling of multiple process modalities with a single model. This can be advantageous for development of operating strategies during process development and assessment of various cell lines from data collected only from batch / fed-batch operation.

A challenge in modeling cell culture systems may be generation of a single system level model that is relevant over multiple modalities. For instance, as also stated above, typically separate models (or at least model coefficients) may be identified for exponential growth versus stationary phases and transferability of models between fed-batch and media exchange is often not contemplated. The modeling of unmeasured lysed cell concentration state ($x_l$ from equation (4)) may enable identification of a single model for use in exponential growth, stationary in fed-batch operation to media exchange, thus enabling identification of a single model that is transferable between metabolic phases and fed-batch versus media exchange operations.

Finding the optimal set of coefficients may be a challenging global optimization problem. Some of the many challenges include:

high correlation between the coefficients;
lack of model validation criteria; and
lack of methods to determine significance of coefficients. With linear orthogonal systems, classical statistical methods for determining significance of parameters can be applied. On the other hand, non-linear systems such as the Monod model as described above do not have equivalent straightforward methods.

## Model Parameter Identification and Simulation

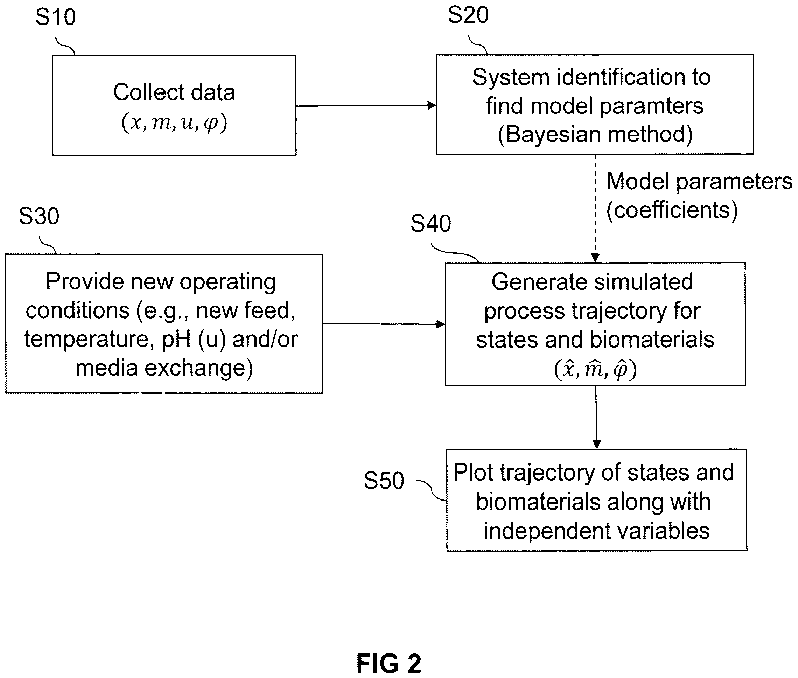

**Fig. 2** shows an exemplary flow of simulating process trajectories for a new set of operating conditions (u). In particular, Fig. 2 shows an exemplary use of the model where model parameters (e.g. coefficients) are identified from a set of data and process trajectories are simulated for a new set of operating conditions (u). The model may be identified from batch / fed-batch operation and simulated for a media exchange set of operating conditions.

The exemplary flow shown in Fig. 2 may be performed by the computing device 20 shown in Fig. 1.

Referring to Fig. 2, data with respect to at least one operation of a cell culture process may be collected (step S10). The collected data may include values of the measurable parameters including one or more operating conditions. The one or more operating conditions may be represented by one or more independent variables (u) and include one or more of: fresh media feed flow rate, fresh media composition, cell bleed flow rate, harvest flow rate, temperature, pH, dissolved oxygen, agitation rate. Further, in some examples, the collected data may include measurable state values such as values of the viable cell concentration $x_v$, the dead cell concentration $x_d$, the viability $x_{viab}$, concentration $m_i$ of the $i^{th}$ metabolite and/or concentration $m_{f,i}$ of the $i^{th}$ metabolite in the fresh feed flow (i = 1, 2, ..., **N,** where **N** may be the number of the metabolites). The collected data may further include the value $\varphi_b$ of a biomaterial. The biomaterial may be used as a toxin.

Accordingly, in step S10, the computing device 20 may obtain measurable parameter values that are measured with respect to at least one operation of a cell culture process carried out at the upstream process system 1 shown in Fig. 1.

The collected data may be used for system identification to find model parameters (e.g. coefficients) with a Bayesian method (step S20). In other words, the computing device 20 may estimate, using Bayesian inference with the measurable parameter values obtained in step S10, values of unmeasurable parameters in a model of the cell culture process. The unmeasurable parameters may be considered as coefficients of the model to be estimated. The model may describe the cell culture process with coupled ordinary differential equations including, e.g., equations (3) to (5).

Estimation of the coefficients may be achieved within a Bayesian framework. From the probabilistic model of the system described herein, a posterior distribution of these parameters may be computed. Hence, as a result, values corresponding to the maximum in the posterior distribution of these coefficients as well as a credible region within which values of coefficients fall with a particular probability range may be obtained.

Considering $X(t)$ as the vector of values describing the Monod growth kinetic models at time $t$, the coupled ordinary differential equations (ODEs) can be written as in equation (17).

$$\frac{dX(t)}{dt} = f(X(t), \beta) \qquad (17)$$

The system may be composed of $K$ coupled ODEs (e.g., the state vector) and $\beta$ may be the parameter vector to be inferred. f may be one or more functions that describe the dynamic of the system (e.g., equations (3) to (5)). The initial conditions, $\mathbf{x}_0$, may or may not be known, hence they may have to be estimated as well.

Consider a set of noisy batch experiments that produced the set of observations $Y$ observed at different time points for the $K$ states. The likelihood $p(Y|X)$ where $X$ is the shortcut for $X(\beta, \mathbf{x}_0)$ can be obtained. The exact definition of the likelihood may depend on how the noise generated by the experiments is modeled. If the noise is considered normally distributed around the most likely value of X, the standard deviation of the normal distribution around the most likely value may need to be taken into account. The value of the standard deviation may also be time dependent. For example, the value of the standard deviation might rise with time since the ODE is further away from the initial condition, the more likely experimental error may occur.

Hence, combining the likelihood with a prior distribution $p(\beta)$ on the parameters, the posterior distribution may be obtained as the following equation (18).

$$p(\beta|Y) = \frac{p(Y|X)p(\beta)}{Z} \qquad (18)$$

The prior distribution may describe what is known concerning the parameter(s) to be estimated. For instance, if the parameters are known to be within a certain range, a uniform distribution between the boundary of the range may be used as a prior distribution. Other prior distribution may be applied, depending on the prior knowledge that is available on the kinetic model parameters. Z may be the marginal likelihood that is usually intractable. For this reason, numerical approximation is often used to produce inference. These approximations may require the usage of Monte Carlo Markov Chain (MCMC) in order to simulate a large number of samples. Progress in sampling methodology as well as continuous progress in computing power available have made accessible such computation to be performed. The estimation of the parameters may be achieved with Monte-Carlo methods such as Nested Sampling, No-U-Turn Sampler (NUTS) but not only.

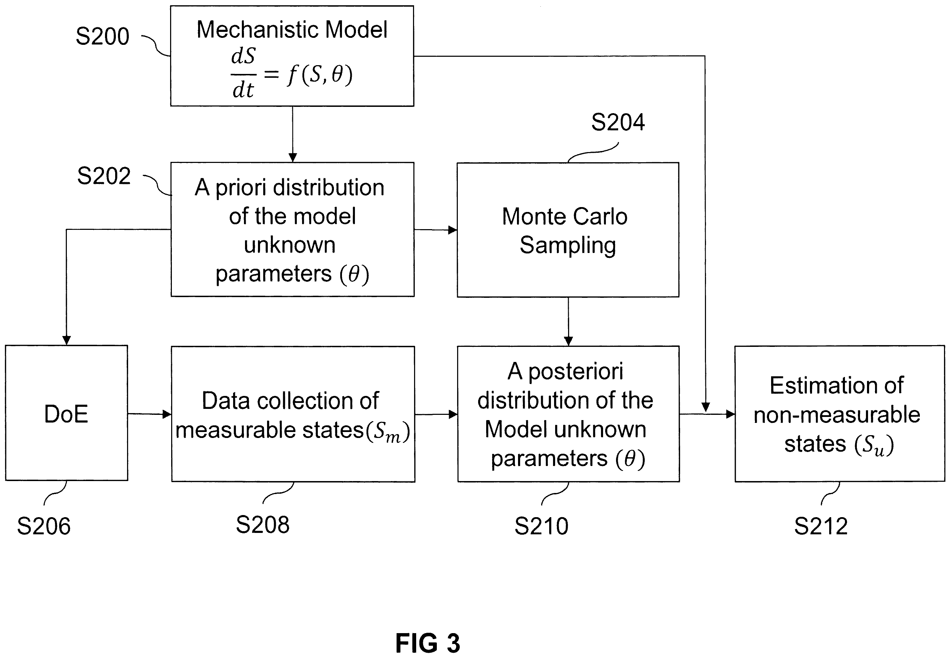

**Fig. 3** shows an exemplary workflow of the Bayesian model parameters and unmeasurable state estimation. The exemplary workflow shown in Fig.3 may be an example of detailed flow of step S20 of Fig. 2. Accordingly, the computing device 20 may perform the exemplary workflow shown in Fig. 3 when performing step S20 shown in Fig. 2.

Referring to Fig. 3, a mechanistic model such as the coupled ODEs defined as in equation (17) above may be provided for the Bayesian inference (step S200). For the parameters to be estimated (in other words, unknown model parameters, e.g. the unmeasurable parameters in the present disclosure), a prior distribution may be determined, based on available knowledge concerning the parameters to be estimated (step S202). The prior distribution may be used in the Monte Carlo Sampling as mentioned above (step S204) and for Design of Experiments (DoE) (step S206).

Referring further to Fig. 3, data regarding measurable states may be collected (step S208) in view of DoE in step S206. The measurable states may be understood as the measurable parameters in the present disclosure. For example, the data collected in step S10 of Fig. 2 may correspond to the data collected in step S208 of Fig. 3. With the result of the Monte Carlo Sampling and the data collected in step S208, a posterior distribution of the parameters to be estimated may be obtained (step S210). From the mechanistic model and the posterior distribution of the unknown parameters, values of the unknown parameters, e.g., values of unmeasurable parameters, may be estimated (step S212).

An example of an unknown parameter that represent an unknown state of the system is lysed cell density, or in other words, concentration of lysed cells. The lysed cells are cells that have died and have disintegrated into the bulk fluid. They are usually invisible and unmeasured. The concentration of lysed cells $x_l$ may be an indicator of toxicity inhibiting growth and increasing death rate. In fed-batch operations these lysed cells may accumulate, leading to a shift in metabolic activity. During media exchange, fresh media ($F_f$) may be introduced to the system, a portion of cells may be bled off ($F_b$) and a portion of spent media (e.g. the bulk fluid in the bioreactor that contains metabolic byproducts and lysed cells) may be separated from the cells for harvest ($F_h$). The exchange of fresh media for spent media can reduce the accumulation of lysed cells and associated bioproducts allowing the metabolic activity to return to a state similar to that in fresh media.

The Bayesian parameter estimation as stated above may provide the posterior distribution of the parameters, given the prior knowledge that is available on the parameters and the available data. Hence the contribution of the data to make the posterior distribution more informative may indeed be dependent on the novel information brought forward by the data. Accordingly, an adequate experimental design may need to be carried out in order to create system variations that minimize the correlation between parameter estimates and thus maximize the information. Simulations of the dynamic system in conjunction with possible experimental settings may need to be studied thoroughly in order to create a set of experiment that will maximize the information provided by the data.

Referring again to Fig. 2, after estimating the values of the unmeasurable parameters of the model in step S20, new operating conditions are provided (step S30) and at least one simulated process trajectory for the states and biomaterials in an operation of the cell culture process may be generated (step S40). In other words, after the estimation in step S20, the computing device 20 receives, in step S30, one or more new measurable parameter values relating to the operating conditions of the cell culture process. Further, the computing device 20 simulates, in step S40, the cell culture process using: the model of the cell culture process; the estimated values of the unmeasurable parameters in the model; and the received one or more new measurable parameter values.

Examples of the new operating conditions provided at step S30 include one or more of: fresh media feed flow rate, fresh media composition, cell bleed flow rate, harvest flow rate, temperature, pH, dissolved oxygen, agitation rate.

The states and biomaterials for which the simulated process trajectory or trajectories can be generated at step S40 may include, but are not limited to, the total cells concentration $x_t$, the viable cell concentration $x_v$, the dead cell concentration $x_d$, the concentration $x_l$ of the lysed cells, the viability $x_{viab}$, concentration $m_i$ of the $i^{th}$ metabolite (i = 1, 2, ..., N, where N may be the number of the metabolites) and/or the value $\varphi_b$ of a biomaterial.

Once the simulated process trajectory or trajectories for the states and biomaterials are generated at step S40, the trajectory or trajectories of the states and biomaterials may be provided along with the independent variables representing the new operating conditions (step S50). For example, the computing device 20 may provide for display at least one image in which the simulated process trajectory or trajectories is or are plotted as well as information indicating the values of the new operating conditions received at step S30. In some examples, the plotted trajectory or trajectories and the information indicating the values of the new operating conditions may be displayed on a display device (not shown) of the computing device 20. Alternatively or additionally, the plotted trajectory or trajectories and the information indicating the values of the new operating conditions may be sent from the computing device 20 to a further device other than the computing device 20 and displayed on a display of that device.

After simulating the cell culture process as described above with reference to Figs. 2 and 3, the simulation result(s) is (are) used for determining optimal operating conditions for obtaining a desired cell growth in the cell culture process. The computing device 20 receives information indicating desired cell growth in the cell culture process and determine optimal operating conditions for obtaining the desired cell growth, according to the result(s) of simulating the cell culture process as described above. The information indicating the desired cell growth may be provided by a user using an input device of the computing device (20) or of another device connected to the computing device 20. The determination of optimal operating conditions for obtaining the desired cell growth may be performed, for example, by identifying, from among the simulation results of the cell culture process, the simulated process trajectory which is closest to that for the desired cell growth and determining the operating conditions with which the simulation process trajectory or trajectories is or are generated.

In some circumstances, the determined optimal operating conditions may be provided for display to a user, on a display of the computing device 20 and/or of another device connected to the computing device 20. The user may then operate the upstream process system 1 to carry out the cell culture process under the determined operating conditions. In this way, displaying the determined optimal operating conditions may provide an improved, continued man-machine interaction in operating the upstream process system 1.

Further, in some examples, the determined optimal operating conditions may be used for controlling the upstream process system 1. In these examples, the computing device may generate one or more control signals for controlling the upstream process system 1 to operate under the determined optimal operating conditions and output the generated one or more control signals to the upstream process system 1. The upstream process system 1 may then operate under the determined optimal operating conditions to obtain the desired cell growth in the cell culture process. Accordingly, in these examples, the computing device 20 may implement a method for controlling the cell culture process.

In some circumstances, the model parameter identification and simulation as described above with reference to Figs. 2 and 3 may enable:

- generation of a single system level model that describes growth behavior,

  - over multiple phases in a fed-batch operation, and
  - between fed-batch and media exchange operations; and

- Bayesian approach for parameter identification to provide,

  - a robust global optimization solution,
  - uncertainty in parameter estimates enabling statistical justification for parameter estimates, and
  - estimates of uncertainty for forecasted model predictions.

Further, in some examples, the model parameter identification and simulation as described above with reference to Figs. 2 and 3 may be integrated into a simulation application. The simulation application may be used for one or more of the following:

- Process simulation

  - Growth curves can be simulated for various feed or process variable (e.g., pH, temperature, dissolved oxygen) trajectories.
  - Enabling simulating growth curves for media exchange operations for models calibrated on fed-batch, which may be understood as being similar to a scaling tool.

- Process optimization

  - Using the model within an optimization routine optimal feed and process trajectories can be identified to maximize growth or follow a desired growth trajectory.

- Process control

  - The growth model can be used as part of a model predictive control strategy to adjust feed and process conditions to track a desired growth profile.

## Hardware Configuration

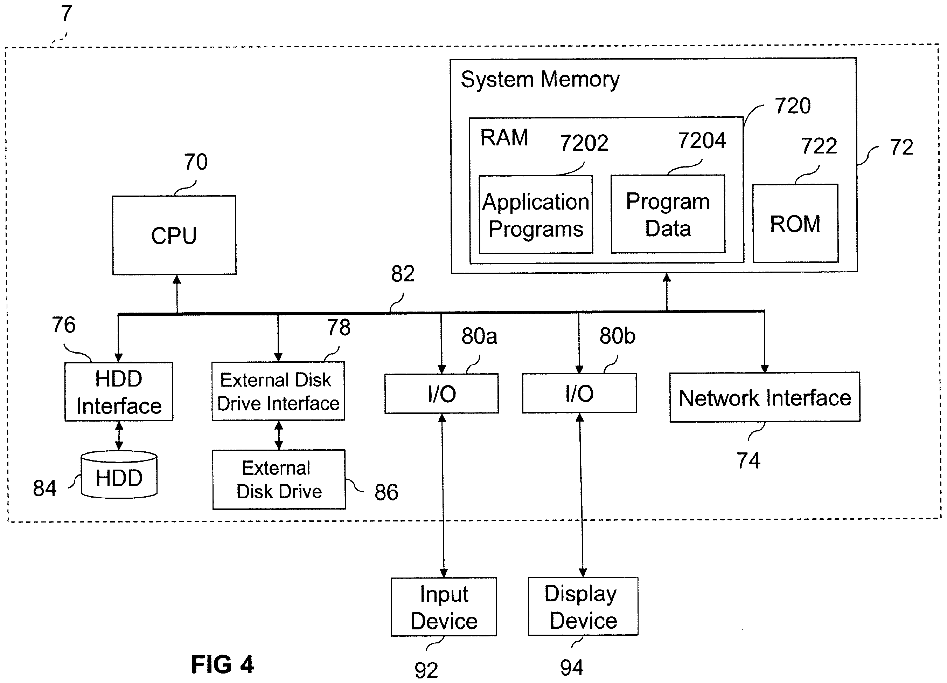

**Fig. 4** shows an exemplary hardware configuration of a computer that may be used to implement at least a part of the system as described above. For example, the computing device 20 shown in Fig. 1 may be implemented with the computer 7 shown in Fig. 4. The computer 7 shown in Fig. 4 includes a central processing unit (CPU) 70, a graphics processing unit (GPU) 88, a system memory 72, a network interface 74, a hard disk drive (HDD) interface 76, an external disk drive interface 78 and input/output (I/O) interfaces 80. These components of the computer are coupled to each other via a system bus 82. The CPU 70 may perform arithmetic, logic and/or control operations by accessing the system memory 72. The system memory 72 may store information and/or instructions for use in combination with the CPU 70. The system memory 72 may include volatile and non-volatile memory, such as a random access memory (RAM) 720 and a read only memory (ROM) 722. A basic input/output system (BIOS) containing the basic routines that helps to transfer information between elements within the computer 7, such as during start-up, may be stored in the ROM 722. The system bus 82 may be any of several types of bus structures including a memory bus or memory controller, a peripheral bus, and a local bus using any of a variety of bus architectures. The CPU 70 may be further connected to one or more sensors (not shown) via one or more corresponding interfaces (not shown) and the bus 82. The sensors may measure physical conditions or states including but not limited to: temperature, pH, pressure, etc. Additionally, the sensors may include other types of measuring or detecting devices including but not limited to imaging devices, microphones, spectral sensors, etc. Controllers may control a physical condition or state including but not limited to: temperature, flux, stirring, etc.

The computer may include a network interface 74 for communicating with other computers and/or devices via a network.

**EP 3 868 860 B1**

Further, the computer may include a hard disk drive (HDD) 84 for reading from and writing to a hard disk (not shown), and an external disk drive 86 for reading from or writing to a removable disk (not shown). The removable disk may be a magnetic disk for a magnetic disk drive or an optical disk such as a CD ROM for an optical disk drive. The HDD 84 and the external disk drive 86 are connected to the system bus 82 by a HDD interface 76 and an external disk drive interface 78, respectively. The drives and their associated computer-readable media provide non-volatile storage of computer-readable instructions, data structures, program modules and other data for the general purpose computer. The data structures may include relevant data for the implementation of the exemplary method and its variations as described herein. The relevant data may be organized in a database, for example a relational or object database.

Although the exemplary environment described herein employs a hard disk (not shown) and an external disk (not shown), it should be appreciated by those skilled in the art that other types of computer readable media which can store data that is accessible by a computer, such as magnetic cassettes, flash memory cards, digital video disks, random access memories, read only memories, and the like, may also be used in the exemplary operating environment.

A number of program modules may be stored on the hard disk, external disk, ROM 722 or RAM 720, including an operating system (not shown), one or more application programs 7202, other program modules (not shown), and program data 7204. The application programs may include at least a part of the functionality as described above.

The computer 7 may be connected to an input device 92 such as mouse and/or keyboard and a display device 94 such as liquid crystal display, via corresponding I/O interfaces 80a and 80b as well as the system bus 82. In case the computer 7 is implemented as a tablet computer, for example, a touch panel that displays information and that receives input may be connected to the computer 7 via a corresponding I/O interface and the system bus 82. Further, in some examples, although not shown in Fig. 4, the computer 7 may further be connected to a printer and/or an imaging device such as a camera, via corresponding I/O interfaces and the system bus 82.

In addition or as an alternative to an implementation using a computer 7 as shown in Fig. 4, a part or all of the functionality of the exemplary embodiments described herein may be implemented as one or more hardware circuits. Examples of such hardware circuits may include but are not limited to: Large Scale Integration (LSI), Reduced Instruction Set Circuits (RISC), Application Specific Integrated Circuit (ASIC) and Field Programmable Gate Array (FPGA).

**Claims**

1. A computer-implemented method for simulating a cell culture process, comprising:

    receiving (S30) one or more measurable parameter values relating to one or more operating conditions of the cell culture process, the one or more operating conditions including one or more of: fresh media feed flow rate, fresh media composition, cell bleed flow rate, harvest flow rate, temperature, pH, dissolved oxygen, agitation rate;
    simulating (S40) the cell culture process using:

        a model of the cell culture process;
        estimated values of unmeasurable parameters in the model; and
        the received one or more measurable parameter values;

    receiving information indicating desired cell growth in the cell culture process; and
    determining, according to a result of said simulating the cell culture process, optimal operating conditions for obtaining the desired cell growth,
    wherein the model of the cell culture process describes the cell culture process with coupled ordinary differential equations including measurable parameters and the unmeasurable parameters, one or more of the measurable parameters relating to said one or more operating conditions of the cell culture process;
    wherein one or more of the unmeasurable parameters relate to lysed cells in the cell culture process;
    wherein said one or more of the unmeasurable parameters include concentration of the lysed cells in the cell culture process and/or concentration of a biomaterial that has a toxic influence on the viable cells, the concentration of the lysed cells being tracked through modeling disintegration of dead cells, production of a toxic biomaterial being modeled as a function of viable cell density,
    wherein the modeling of the disintegration of the dead cells involves cell death rate that is adjusted by the concentration of the lysed cells and/or concentration of the toxic biomaterial; and
    wherein the estimated values of the unmeasurable parameters are estimated using Bayesian inference with measurable parameter values that are measured with respect to at least one operation of the cell culture process.

2. The computer-implemented method according to claim 1, further comprising:

obtaining (S10) the measurable parameter values that are measured with respect to the at least one operation of the cell culture process;

estimating (S20), using Bayesian inference with the obtained measurable parameter values, values of unmeasurable parameters in the model to be used as the estimated values of the unmeasurable parameters in said simulating the cell culture process.

3. The method according to any one of claims 1 or 2, further comprising:

generating one or more control signals for controlling one or more devices that carry out the cell culture process to operate under the determined optimal operating conditions; and

outputting the generated one or more control signals.

4. A computer program product comprising computer-readable instructions that, when loaded and run on a computer, cause the computer to perform the method according to any one of claims 1 to 3.

5. A system for simulating a cell culture process, comprising:

a storage medium (30) storing a model of the cell culture process, the model describing the cell culture process with coupled ordinary differential equations including measurable parameters and unmeasurable parameters, wherein one or more of the measurable parameters relate to one or more operating conditions of the cell culture process and one or more of the unmeasurable parameters relate to lysed cells in the cell culture process, the one or more operating conditions including one or more of: fresh media feed flow rate, fresh media composition, cell bleed flow rate, harvest flow rate, temperature, pH, dissolved oxygen, agitation rate; and

a processor configured to:

receive (S30) one or more measurable parameter values relating to said one or more operating conditions of the cell culture process;

simulate (S40) the cell culture process using:

the model of the cell culture process;

estimated values of the unmeasurable parameters in the model, wherein the estimated values of the unmeasurable parameters are estimated using Bayesian inference with measurable parameter values that are measured with respect to at least one operation of the cell culture process; and

the received one or more measurable parameter values;

receive information indicating desired cell growth in the cell culture process; and

determine, according to a result of said simulating the cell culture process, optimal operating conditions for obtaining the desired cell growth,

wherein said one or more of the unmeasurable parameters include concentration of the lysed cells in the cell culture process and/or concentration of a biomaterial that has a toxic influence on the viable cells, the concentration of the lysed cells being tracked through modeling disintegration of dead cells, production of a toxic biomaterial being modeled as a function of viable cell density,

wherein the modeling of the disintegration of the dead cells involves cell death rate that is adjusted by the concentration of the lysed cells and/or concentration of the toxic biomaterial.

6. The system according to claim 5, wherein the processor is further configured to:

obtain (S10) the measurable parameter values that are measured with respect to the at least one operation of the cell culture process; and

estimate (S20), using Bayesian inference with the obtained measurable parameter values, values of the unmeasurable parameters in the model to be used as the estimated values of the unmeasurable parameters in said simulating the cell culture process.

7. The system according to claim 5 or 6, wherein the processor is further configured to:

generate one or more control signals for controlling one or more devices that carry out the cell culture process to operate under the determined optimal operating conditions; and

output the generated one or more control signals.

**Patentansprüche**

1. Computerimplementiertes Verfahren zum Simulieren eines Zellkulturprozesses, das Folgendes umfasst:

   Empfangen (S30) von einem oder mehreren messbaren Parameterwerten, die sich auf eine oder mehrere Ablaufbedingungen des Zellkulturprozesses beziehen, wobei die eine oder die mehreren Ablaufbedingungen eines oder mehreres von Folgendem beinhalten: einer Frischmedienzuflussrate, einer Frischmedienzusammensetzung, einer Zellleckstromrate, einer Erntestromrate, Temperatur, pH, aufgelöstem Sauerstoff, einer Rührrate;
   Simulieren (S40) des Zellkulturprozesses unter Verwendung von Folgendem:

   einem Modell des Zellkulturprozesses;
   Schätzwerten von unmessbaren Parametern im Modell; und
   dem einen oder den mehreren empfangenen messbaren Parameterwerten;
   Empfangen von Informationen, die ein gewünschtes Zellwachstum im Zellkulturprozess anzeigen; und
   Bestimmen von optimalen Ablaufbedingungen zum Erhalten des gewünschten Zellwachstums gemäß einem Ergebnis des Simulierens des Zellkulturprozesses,
   wobei das Modell des Zellkulturprozesses den Zellkulturprozess mit gekoppelten gewöhnlichen Differenzialgleichungen beschreibt, die messbare Parameter und die unmessbaren Parameter beinhalten, wobei sich einer oder mehrere der messbaren Parameter auf die eine oder die mehreren Ablaufbedingungen des Zellkulturprozesses beziehen;
   wobei sich ein oder mehrere der unmessbaren Parameter auf lysierte Zellen im Zellkulturprozess beziehen;
   wobei der eine oder die mehreren unmessbaren Parameter eine Konzentration von lysierten Zellen im Zellkulturprozess und/oder eine Konzentration eines Biomaterials beinhalten, das einen toxischen Einfluss auf die lebensfähigen Zellen hat, wobei die Konzentration der lysierten Zellen mittels Modellieren eines Zerfalls von toten Zellen verfolgt wird, wobei eine Produktion von toxischem Biomaterial in Abhängigkeit von einer Dichte von lebensfähigen Zellen modelliert wird,
   wobei das Modellieren des Zerfalls der toten Zellen eine Zelltodesrate einschließt, die durch die Konzentration der lysierten Zellen und/oder die Konzentration von toxischem Biomaterial angepasst werden kann; und
   wobei die geschätzten Werte der unmessbaren Parameter unter Verwendung einer Bayesschen Inferenz mit messbaren Parameterwerten geschätzt werden, die mit Bezug auf mindestens einen Ablauf des Zellkulturprozesses gemessen werden.

2. Computerimplementiertes Verfahren nach Anspruch 1, das ferner Folgendes umfasst:

   Erhalten (S10) der messbaren Parameterwerte, die mit Bezug auf den mindestens einen Ablauf des Zellkulturprozesses gemessen werden;
   Schätzen (S20) von Werten von unmessbaren Parametern in dem Modell, die beim Simulieren des Zellkulturprozesses als die geschätzten Werte der unmessbaren Parameter zu verwenden sind, unter Verwendung der Bayesschen Inferenz mit den erhaltenen messbaren Parameterwerten.

3. Verfahren nach einem der Ansprüche 1 oder 2, das ferner Folgendes umfasst:

   Erzeugen von einem oder mehreren Steuersignalen zum Steuern von einer oder mehreren Vorrichtungen, die den Zellkulturprozess umsetzen, um unter den bestimmten optimalen Ablaufbedingungen abzulaufen; und
   Ausgeben des einen oder der mehreren erzeugten Steuersignale.

4. Computerprogrammprodukt, das computerlesbare Anweisungen umfasst, die, wenn sie in einen Computer geladen und von demselben ausgeführt werden, den Computer veranlassen, das Verfahren nach einem der Ansprüche 1 bis 3 durchzuführen.

5. System zum Simulieren eines Zellkulturprozesses, das Folgendes umfasst:

   ein Speichermedium (30), in dem ein Modell des Zellkulturprozesses gespeichert ist, wobei das Modell den Zellkulturprozess mit gekoppelten gewöhnlichen Differenzialgleichungen beschreibt, die messbare Parameter und unmessbare Parameter beinhalten, wobei sich ein oder mehrere der messbaren Parameter auf eine oder mehrere Ablaufbedingungen des Zellkulturprozesses beziehen und ein oder mehrere der unmessbaren Parameter sich auf lysierte Zellen im Zellkulturprozess beziehen, wobei die eine oder die mehreren Ablaufbedingungen eines oder mehreres von Folgendem beinhalten: einer Frischmedienzuflussrate, einer Frischmedienzusammensetzung, einer Zellleckstromrate, einer Erntestromrate, Temperatur, pH, aufgelöstem Sauerstoff, einer Rührrate; und

einen Prozessor, der zu Folgendem ausgelegt ist:

Empfangen (S30) von einem oder mehreren messbaren Parameterwerten, die sich auf die eine oder die mehreren Ablaufbedingungen des Zellkulturprozesses beziehen;

Simulieren (S40) des Zellkulturprozesses unter Verwendung von Folgendem:

dem Modell des Zellkulturprozesses;

geschätzten Werten der unmessbaren Parameter im Modell, wobei die geschätzten Werte der unmessbaren Parameter unter Verwendung einer Bayesschen Inferenz mit messbaren Parameterwerten geschätzt werden, die mit Bezug auf mindestens einen Ablauf des Zellkulturprozesses gemessen werden; und

dem einen oder den mehreren empfangenen messbaren Parameterwerten;

Empfangen von Informationen, die ein gewünschtes Zellwachstum im Zellkulturprozess anzeigen; und

Bestimmen von optimalen Ablaufbedingungen zum Erhalten des gewünschten Zellwachstums gemäß einem Ergebnis des Simulierens des Zellkulturprozesses,

wobei der eine oder die mehreren unmessbaren Parameter eine Konzentration von lysierten Zellen im Zellkulturprozess und/oder eine Konzentration eines Biomaterials beinhalten, das einen toxischen Einfluss auf die lebensfähigen Zellen hat, wobei die Konzentration der lysierten Zellen mittels Modellieren eines Zerfalls von toten Zellen verfolgt wird, wobei eine Produktion von toxischem Biomaterial in Abhängigkeit von einer Dichte von lebensfähigen Zellen modelliert wird,

wobei das Modellieren des Zerfalls der toten Zellen eine Zelltodesrate einschließt, die durch die Konzentration der lysierten Zellen und/oder die Konzentration von toxischem Biomaterial angepasst werden kann.

6. System nach Anspruch 5, wobei der Prozessor ferner zu Folgendem ausgelegt ist:

Erhalten (S10) der messbaren Parameterwerte, die mit Bezug auf den mindestens einen Ablauf des Zellkulturprozesses gemessen werden; und

Schätzen (S20) von Werten der unmessbaren Parameter in dem Modell, die beim Simulieren des Zellkulturprozesses als die geschätzten Werte der unmessbaren Parameter zu verwenden sind, unter Verwendung der Bayesschen Inferenz mit den erhaltenen messbaren Parameterwerten.

7. System nach Anspruch 5 oder 6, wobei der Prozessor ferner zu Folgendem ausgelegt ist:

Erzeugen von einem oder mehreren Steuersignalen zum Steuern von einer oder mehreren Vorrichtungen, die den Zellkulturprozess umsetzen, um unter den bestimmten optimalen Ablaufbedingungen abzulaufen; und

Ausgeben des einen oder der mehreren erzeugten Steuersignale.

**Revendications**

1. Procédé mis en œuvre par ordinateur pour simuler un processus de culture cellulaire, comprenant :

la réception (S30) d'une ou plusieurs valeurs de paramètres mesurables se rapportant à une ou plusieurs conditions de fonctionnement du processus de culture cellulaire, les une ou plusieurs conditions de fonctionnement comportant un ou plusieurs parmi : un débit d'alimentation en milieu frais, une composition de milieu frais, un débit de perte de cellules, un débit de récolte, une température, le pH, de l'oxygène dissous, un taux d'agitation ;

la simulation (S40) du processus de culture cellulaire en utilisant :

un modèle du processus de culture cellulaire ;

des valeurs estimées des paramètres non mesurables dans le modèle ; et

les une ou plusieurs valeurs de paramètres mesurables reçues ;

la réception d'informations indiquant une croissance cellulaire souhaitée dans le processus de culture cellulaire ; et

selon un résultat de ladite simulation du processus de culture cellulaire, la détermination de conditions de fonctionnement optimales pour obtenir la croissance cellulaire souhaitée,

dans lequel le modèle du processus de culture cellulaire décrit le processus de culture cellulaire avec des équations différentielles ordinaires couplées comportant des paramètres mesurables et les paramètres non mesurables, un ou plusieurs des paramètres mesurables étant liés auxdites une ou plusieurs conditions de fonctionnement du processus de culture cellulaire ;

dans lequel un ou plusieurs des paramètres non mesurables se rapportent à des cellules lysées dans le processus de culture cellulaire ;

dans lequel lesdits un ou plusieurs des paramètres non mesurables comportent la concentration des cellules lysées dans le processus de culture cellulaire et/ou la concentration d'un biomatériau qui a une influence toxique sur les cellules viables, la concentration des cellules lysées étant suivie par modélisation de la désintégration des cellules mortes, la production d'un biomatériau toxique étant modélisée en fonction d'une densité de cellules viables,

dans lequel la modélisation de la désintégration des cellules mortes implique un taux de mort cellulaire qui est ajusté par la concentration des cellules lysées et/ou la concentration du biomatériau toxique ; et

dans lequel les valeurs estimées des paramètres non mesurables sont estimées en utilisant l'inférence bayésienne avec des valeurs de paramètres mesurables qui sont mesurées par rapport à au moins une opération du processus de culture cellulaire.

2. Procédé mis en œuvre par ordinateur selon la revendication 1, comprenant en outre :

l'obtention (S10) des valeurs de paramètres mesurables qui sont mesurées par rapport à l'au moins une opération du processus de culture cellulaire ;

en utilisant l'inférence bayésienne avec les valeurs de paramètres mesurables obtenues, l'estimation (S20) de valeurs de paramètres non mesurables dans le modèle à utiliser comme valeurs estimées des paramètres non mesurables dans ladite simulation du processus de culture cellulaire.

3. Procédé selon l'une quelconque des revendications 1 ou 2, comprenant en outre :

la génération d'un ou plusieurs signaux de commande pour commander un ou plusieurs dispositifs qui effectuent le processus de culture cellulaire pour fonctionner dans les conditions de fonctionnement optimales déterminées ; et

la sortie des un ou plusieurs signaux de commande générés.

4. Produit de programme informatique comprenant des instructions lisibles par ordinateur qui, lorsqu'elles sont chargées et exécutées sur un ordinateur, amènent l'ordinateur à réaliser le procédé selon l'une quelconque des revendications 1 à 3.

5. Système pour simuler un processus de culture cellulaire, comprenant :

un support de stockage (30) stockant un modèle du processus de culture cellulaire, le modèle décrivant le processus de culture cellulaire avec des équations différentielles ordinaires couplées comportant des paramètres mesurables et des paramètres non mesurables, dans lequel un ou plusieurs des paramètres mesurables se rapportent à une ou plusieurs conditions de fonctionnement du processus de culture cellulaire, et un ou plusieurs des paramètres non mesurables se rapportent à des cellules lysées dans le processus de culture cellulaire, les une ou plusieurs conditions de fonctionnement comportant un ou plusieurs parmi : un débit d'alimentation en milieu frais, une composition de milieu frais, un débit de perte de cellules, un débit de récolte, une température, le pH, de l'oxygène dissous, un taux d'agitation ; et

un processeur configuré pour :

recevoir (S30) une ou plusieurs valeurs de paramètres mesurables se rapportant auxdites une ou plusieurs conditions de fonctionnement du processus de culture cellulaire ;

simuler (S40) le processus de culture cellulaire en utilisant :

le modèle du processus de culture cellulaire ;

des valeurs estimées des paramètres non mesurables dans le modèle, dans lequel les valeurs estimées des paramètres non mesurables sont estimées en utilisant l'inférence bayésienne avec des valeurs de paramètres mesurables qui sont mesurées par rapport à au moins une opération du processus de culture cellulaire ; et

les une ou plusieurs valeurs de paramètres mesurables reçues ;

recevoir des informations indiquant une croissance cellulaire souhaitée dans le processus de culture cellulaire ; et

selon un résultat de ladite simulation du processus de culture cellulaire, déterminer des conditions de fonctionnement optimales pour obtenir la croissance cellulaire souhaitée,

dans lequel lesdits un ou plusieurs des paramètres non mesurables comportent la concentration des cellules lysées dans le processus de culture cellulaire et/ou la concentration d'un biomatériau qui a une influence toxique sur les cellules viables, la concentration des cellules lysées étant suivie par modélisation de la désintégration des cellules mortes, la production d'un biomatériau toxique étant modélisée en fonction d'une densité de cellules viables,

dans lequel la modélisation de la désintégration des cellules mortes implique un taux de mort cellulaire qui est ajusté par la concentration des cellules lysées et/ou la concentration du biomatériau toxique.

6. Système selon la revendication 5, dans lequel le processeur est en outre configuré pour :

obtenir (S10) les valeurs de paramètres mesurables qui sont mesurées par rapport à l'au moins une opération du processus de culture cellulaire ; et

en utilisant l'inférence bayésienne avec les valeurs de paramètres mesurables obtenues, estimer (S20) des valeurs de paramètres non mesurables dans le modèle à utiliser comme valeurs estimées des paramètres non mesurables dans ladite simulation du processus de culture cellulaire.

7. Système selon la revendication 5 ou 6, dans lequel le processeur est en outre configuré pour :

générer un ou plusieurs signaux de commande pour commander un ou plusieurs dispositifs qui effectuent le processus de culture cellulaire pour fonctionner dans les conditions de fonctionnement optimales déterminées ; et

délivrer les un ou plusieurs signaux de commande générés.

EP 3 868 860 B1

**FIG 1**

**S10** Collect data $(x, m, u, \varphi)$

**S20** System identification to find model paramters (Bayesian method)

Model parameters (coefficients)

**S30** Provide new operating conditions (e.g., new feed, temperature, pH (u) and/or media exchange)

**S40** Generate simulated process trajectory for states and biomaterials $(\hat{x}, \hat{m}, \hat{\varphi})$

**S50** Plot trajectory of states and biomaterials along with independent variables

**FIG 2**

**FIG 3**

FIG 4

EP 3 868 860 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **SARANTOS KYRIAKOPOULOS et al.** Kinetic Modeling of Mammalian Cell Culture Bioprocessing: The Quest to Advance Biomanufacturing. *BIOTECHNOLOGY JOURNAL*, 14 November 2017, ISSN 1860-6768
- Metabolic flux-based modeling of mAb production during batch and fed-batch operations. **PENNY DORKA et al.** BIOPROCESS AND BIOSYSTEMS ENGINEERING. SPRINGER, 17 June 2008, vol. 32, 183-196
- **CRAVEN STEPHEN et al.** Glucose concentration control of a fed-batch mammalian cell bioprocess using a nonlinear model predictive controller. *JOURNAL OF PROCESS CONTROL*, 21 March 2014, vol. 24 (4), ISSN 0959-1524, 344-357
- Bioprocess optimization under uncertainty using ensemble modeling. **LIU YANG et al.** JOURNAL OF BIOTECHNOLOGY. ELSEVIER, 27 January 2017, vol. 244, 34-44